# EUROPEAN PATENT SPECIFICATION

(11) **EP 3 944 835 B1**
(45) Date of publication and mention of the grant of the patent: **12.03.2025**
(21) Application number: 21188645.2
(22) Date of filing: 30.07.2021
(51) Int. Cl.: A61B 34/20, A61B 90/00

(54) **SYSTEMS AND METHODS FOR TRACKING A SURGICAL DEVICE**
SYSTEME UND VERFAHREN ZUR NACHVERFOLGUNG EINES MEDIZINPRODUKTS
SYSTÈMES ET PROCÉDÉS DE SUIVI D'UN DISPOSITIF CHIRURGICAL

(30) Priority: 31.07.2020 US 202063059805 P; 23.07.2021 US 202117383980
(43) Date of publication of application: 02.02.2022
(73) Proprietor: Biosense Webster (Israel) Ltd., Yokneam 2066717 (IL)
(72) Inventor: HOD, Uriel, Yokneam (IL); RACHLI, Noam, Hadera (IL); LEVI, Moran, Yokneam (IL); WOLFSON, Helen, Yokneam (IL); BUSTAN, Itamar, Yokneam (IL)
(74) Representative: Carpmaels & Ransford LLP

(56) References cited:
- US-A1- 2020 107 701
- US-A1- 2020 107 886
- US-A1- 2020 242 339

## Description

### FIELD OF INVENTION

The invention is defined by the appended claims, and relates generally to the registration of different coordinate systems, and specifically to the registration of different coordinate systems for a surgical procedure.

### BACKGROUND

In an invasive surgical procedure, including a minimally invasive procedure, it is typically necessary to track a surgical device, such as a catheter, within a patient that is not directly visible to a physician performing the procedure. Typically, a computerized tomography (CT) image, such as from fluoroscopy or magnetic resonance imaging (MRI), of the patient is available to the physician.

US 2020/0242339 A1 describes an apparatus, including a patient tracker, attached to a subject, having magnetic field sensors and optical landmarks with known spatial relationships to each other. A camera acquires a 3D optical image, in a first frame of reference (FOR), of the subject's face. A magnetic radiator assembly generates magnetic fields at the subject's head, thereby defining a second FOR. US 2020/0242339 A1 also describes a processor which: processes field sensor signals to acquire location coordinates of the sensors in the second FOR; segments a tomographic image of the subject, having a third FOR, to identify the subject's face in the third FOR; computes a first transformation between the third and first FORs to map the tomographic face image to the 3D optical image; maps the optical landmarks to the third FOR; maps the respective location coordinates of the sensors to the first FOR; and computes a second transformation between the second FOR and the third FOR.

One method used for tracking surgical devices within a patient is the TruDi^{™} electromagnetic image-guided navigation system, produced by Acclarent, Inc., of 33 Technology Drive, Irvine, CA 92618 USA. In this system, alternating magnetic fields are transmitted, from fixed transmitters external to the patient, so as to pass through the patient. If a procedure is being performed on a patient's head, such as ear, nose and throat (ENT) procedure, then the fixed magnetic field transmitters may be placed around the patent's head. As part of the procedure, the surgical device is placed in the middle of the calibration chamber, and three orthogonal fields may be applied. A sensor, typically a single or multiple axis coil, is attached to the surgical device and inserted into the patient and takes voltage measurements. A processor records the currents generated by the fields traversing the sensor. The processor analyzes the currents so as to determine both the position and the orientation of the sensor in an electromagnetic frame of referenced defined by the fixed transmitters.

Current magnetic based location detection systems may also utilize flexible sensors located on the surgical device in combination with an algorithm or processor to estimate the position, shape, and size of the surgical device, based on voltage measurements taken by the sensors. Flexible sensors typically consist of single-axis sensors (SAS) with 9 transmitters and 1 sensor, for a total of 9 measurements taken at a single point. A tri-axial sensor (TAS), comprising 3 sensors, with 9 transmitters and 2 coils, collects a total of 27 voltage measurements at a single point. When the surgical device is navigated or advanced, additional points may be collected within a short amount of time.

The registration of the CT image with a three-dimensional (3D) camera image is a preliminary step in the TruDi^{™} navigation procedure. Registration involves the localization of the surgical device relative to the registered CT image. In other words, by registering or matching up, the CT image with a 3D image and magnetic coordinates, the location of a surgical device being tracked by an electromagnetic tracking system may be accurately determined and displayed on said 3D camera image, CT image, or combination thereof. In some instances, it may be beneficial to provide an operator with a 3D view of surface of anatomical structures in the head of a patient. After completing the registration of the 3D image with the CT image and the 3D image with the magnetic coordinates, the CT image may then be registered with the magnetic coordinates. Current systems for registering the CT image with the 3D camera image require equipment such as a patient tracker to be attached to the patient's head and certain measures must be taken to account for the patient tracker during registration. It would be desirable to have a streamlined system and method for registering the CT image with the 3D camera image.

### SUMMARY

Systems, methods, and devices for registering a 3D image of a patient with magnetic coordinates are disclosed. A tri-axial sensor (TAS) is added to the 3D camera. The location and orientation of the TAS sensor may be determined based on the known magnetic fields that are applied by a magnetic field transmitter. The camera coordinate system may then be transferred to the magnetic coordinate system. After completing the registration of the 3D image with a CT image and the 3D image with the magnetic coordinates, the CT image may then be registered with the magnetic coordinates. By registering the CT image with the magnetic coordinates, the location of a catheter being tracked by the electromagnetic tracking system may be accurately shown on a display of the CT image, optical image, or combination thereof. The invention is defined by the appended claims.

### BRIEF DESCRIPTION OF THE DRAWING

FIG. 1 is a schematic illustration of a registration system, according to an embodiment;
FIGs. 2A and 2B are schematic figures illustrating a patient tracker used in the system of FIG. 1, according to an embodiment;
FIG. 3 is a flowchart diagram of a method for executing a registration algorithm, according to an embodiment;
FIG. 4 is a flowchart diagram of a method for executing a registration algorithm, according to another embodiment;
FIG. 5 is a schematic illustration of a 3D scatter plot corresponding to an optical image of a patient positioned in a registration system, according to an embodiment; and
FIGs. 6A-6C are schematic diagrams illustrating the mapping of a 3D scatter plot corresponding to an optical image to a CT image.

### DETAILED DESCRIPTION

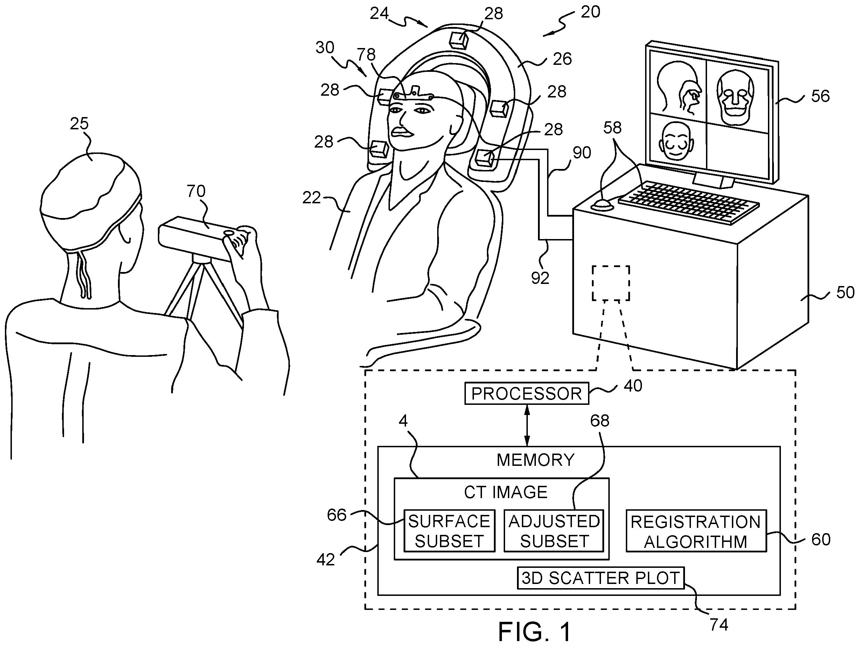

FIG. 1 is a schematic illustration of a registration system 20, according to an embodiment of the present disclosure.

The medical procedure undergone by the patient is assumed to comprise tracking of an surgical device, such as a catheter, which is inserted into the patient by a medical professional 25. The tracking is provided by an electromagnetic tracking system 24, described in more detail below.

The electromagnetic tracking system comprises a magnetic radiator assembly 26, which is positioned around the patient's head. Assembly 26 comprises magnetic field transmitters 28, which are fixed in position and transmit alternating sinusoidal magnetic fields into a region 30, where the head of the patient 22 is located. By way of example, magnetic field transmitters 28 of assembly 24 are arranged in an approximately horseshoe shape around the head of the patient 22. However, alternate configurations for the radiators of assembly 26 will be apparent to those having ordinary skill in the art, and all such configurations are assumed to be comprised within the scope of the present invention.

A magnetic sensor, herein assumed to be a coil, is attached to the surgical device being tracked within the patient 22. The attached coil generates electrical signals in response to the alternating magnetic fields traversing the coil, and these signals are transferred to a system processor 40. The processor 40 is configured to process the signals so as to derive location and orientation values for the sensor. Other elements of the system 20, including magnetic transmitters 28, are controlled by the system processor 40.

The TruDi^{™} system referred to above uses a tracking system similar to that described herein for finding the location and orientation of a coil in a region irradiated by magnetic fields.

The processor 40 uses software stored in a memory 42 to operate system 20. The software may be downloaded to the processor 40 in electronic form, over a network, for example, or it may, additionally or alternatively, be provided and/or stored on non-transitory tangible media, such as magnetic, optical or electronic memory. The processor 40 uses the software to analyze the signals received from the magnetic sensors. Software for a registration algorithm 60 in implementing registration system 20, which executed by the processor 40, is also stored in memory 42. Registration algorithm 60 is described in more detail below.

The processor 40 may be mounted in a console 50, which comprises operating controls 58 that typically include a keypad and/or pointing device such as a mouse or trackball. The console 50 connects to the radiators via a cable 92 and/or wirelessly. The medical professional 25 may use operating controls 58 to interact with the processor while performing the medical procedure described above. While performing the procedure, the processor may present results of the procedure on a screen 56. The presentation of the results of the procedure on the screen 56 allows for a medical professional 25 using the system to visualize the precise location of surgical device, such as a catheter, relative to a CT image of the patient.

As described above, the electromagnetic tracking system 24 is able to track the position and orientation of a magnetic sensor in region 30, by virtue of the magnetic fields transmitted into the region from magnetic transmitters 28. It will be understood that the position and orientation derived for system 24 is with reference to a frame of reference (FOR) of the magnetic system, as defined by the positions of magnetic transmitters 28. In order for the tracking of the sensor to be useful, the magnetic system FOR needs to be registered with the FOR for an image of the patient 22 that is stored in memory 42. Subsets 66 and 68 of image 64, described further below, are also stored in memory 42.

While the CT image may typically comprise a magnetic resonance imaging (MRI) image or a fluoroscopic image, in the description herein the image is assumed to comprise, by way of example, a fluoroscopic CT image.

The medical professional 25 uses a three-dimensional (3D) camera 70 to capture a 3D optical image of the face of patient 22. In some embodiments, the camera 70 is a RealSense 3D camera, produced by Intel Corporation of Santa Clara, California. The 3D camera 70 may comprise at least one optical sensor. In some embodiments, the 3D camera 70 may comprise two, separate optical sensors. The 3D optical image comprises a set of optical voxels, each voxel having three Cartesian coordinates as well as color, typically red, green, blue (RGB) values. The set of optical voxels is herein also termed a 3D scatter plot 74, and the optical voxels of scatter plot 74 are stored in memory 42.

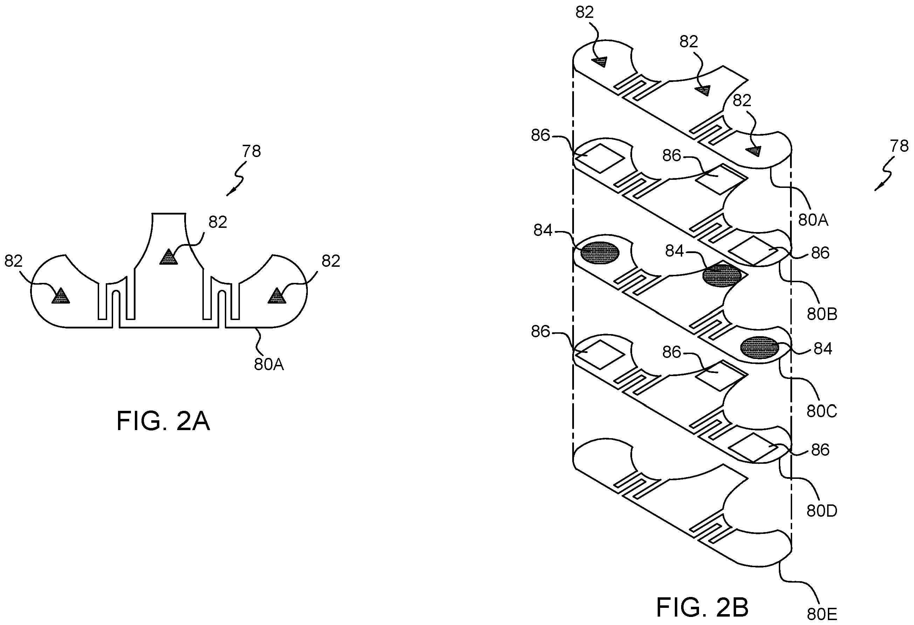

For the registration implemented by the system 20, a patient tracker 78 is positioned on patient 22. The patient tracker 78 is described with reference to FIGs. 2A and 2B, described below.

FIGs. 2A and 2B are schematic figures illustrating the patient tracker 78 according to an embodiment. Patient tracker 78 is formed as a substantially planar sheet, and FIG. 2A illustrates a view of the tracker, as seen by the camera 70. i.e., after the tracker has been positioned on the patient 22. FIG. 2B is an exploded view of the tracker.

In some embodiments, the patient tracker 78 is constructed of five laminar sheets 80A, 80B, 80C, 80D, and 80E, all sheets having substantially the same shape, and being bonded together. Sheet 80A is an upper sheet, also shown in FIG. 2A, and incorporated in the sheet is a plurality of optically identifiable landmarks 82. By way of example, sheet 80A comprises three optical landmarks 82. However, other embodiments may comprise other numbers of landmarks.

Sheet 80C is an intermediary laminar sheet, typically formed from a flexible insulating material, upon which are formed, typically by printing, planar conducting coils 84 in the form of conductive spirals. Coils 84 act as electromagnetic sensors. These are the same number of coils 84 as landmarks 82, and each coil is located on sheet 80C so that it is in a known spatial relationship with respective landmark 82. By way of example, each coil 84 is located to be directly aligned with a respective landmark 82 when the sheets of the tracker are bonded together. However, other embodiments may be formed with different known spatial relationships between the coils and the landmarks. For example, coils and landmarks may be offset by known spatial amounts.

A cable 90 (illustrated in FIG. 1) connects coils 84 to processor 40. Connections of coils 84 to the cable are not shown in FIGs. 2A and 2B for simplicity.

Sheet 80E is a lower laminar sheet formed from biocompatible adhesive, and it is this sheet that contacts patient 22 during operation of system 20.

Sheets 80B and 80D are intermediate laminar sheets, formed of conductive material, so as to act as electrical shields for coils 84. Within sheets 80B are non-conductive regions 86 aligned with coils 84. The presence of the non-conductive regions 86 enable the coils to operate correctly. In some embodiments, the non-conductive regions 86 are openings.

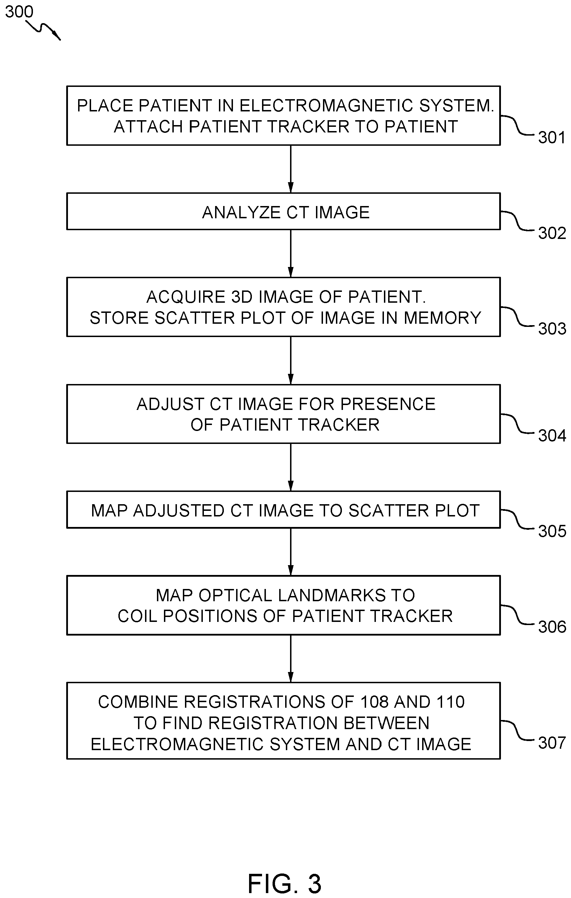

FIG. 3 is a flowchart diagram of a method 300 for executing registration algorithm 60, according to an embodiment. Method 300 may be performed on the registration system 20 illustrated in FIG. 1.

At 301, the electromagnetic tracking system 24 is activated, and the head of patient 22 is placed within region 30 of the system. Patient tracker 78 is attached to the forehead of the patient, using biocompatible adhesive sheet 80E, and so that optical landmarks 82 are uppermost and are visible. Cable 90 is connected between the patient tracker and processor 40, and the processor 40 may be activated to acquire signals conveyed by the cable from coils 84. The processor analyzes the signals to calculate the positions of the coils in the FOR defined by magnetic transmitters 28. If the calculated positions are found to be within an expected part of region 30, processor 40 may provide an indication that the electromagnetic tracking system 24 is operating correctly to medical professional 25. An example indication of the electromagnetic tracking system 24 operating correctly is the processor sending a notification that is displayed on screen 56.

At 302, the processor 40 may analyze a CT image of the head of the patient stored in memory 42. In some embodiments, the processor 40 may analyze the image to identify a subset of CT voxels of the stored image corresponding to surface features of the head of the patient, and the subset may be stored as surface subset 66.

At 303, medical professional 25 activates the 3D camera 70 to acquire a 3D optical image of the face of patient 22, and the acquired image is stored as scatter plot 74 in memory 42. It will be understood that the image acquired by the 3D camera 70 includes an image of patient tracker 78 that is on the face of the patient.

Any suitable algorithm may be used to find the transformation that best maps surface subset of CT voxels 66 to the optical voxels of 3D scatter plot 74. For example, any cloud point matching algorithm, such as robust point matching and kernel correlation, may be used. In some embodiments, an Iterative Closest Point (ICP) algorithm may be used. However, up to 303, there is a known difference in the two sets of voxels, since an image of the patient tracker is present in scatter plot 74 but is not present in CT voxel subset 66.

At 304, the absence of an image of the patient tracker in CT voxel subset 66 is compensated for by adding an image of the patient tracker to the CT voxel subset. The addition may be implemented by presenting an image of the CT voxel subset to the medical professional 25 on screen 56, allowing the professional to overlay an image of the patient tracker on the presented image, and storing the combined image as an adjusted CT voxel subset 68.

Alternatively, at 304, adjusted subset 68 is derived from CT voxel subset 66 by professional 25 selecting portions of subset 66 that do not include the patient tracker image. The medical professional 25 may perform the selection on an image of subset 66 presented on screen 56, and the selected portions are stored as adjusted CT voxel subset 68.

At 305, the processor 40 maps adjusted CT voxel subset 68 to the voxels of scatter plot 74. If at 304 the adjusted CT subset includes an image of the patient tracker, then the mapping may be performed for all the voxels of the two sets. Alternatively, if 304 is implemented by selecting portions of subset 66 that do not include the patient tracker image, the processor 40 makes a corresponding selection in the voxels of scatter plot 74, and the mapping is performed between the selected sets of voxels.

The mapping provides a registration between the FOR of the CT image of the patient 22 and the FOR of the optical image of the patient. The processor 40 may quantify the registration as a first transformation matrix M[CT-OPT] which may be used to transform entities in one of the frames of reference to the other FOR.

At 306, the processor 40 uses the known spatial relationship between optical landmarks 82 and coils 84 to perform a mapping between the locations of the landmarks in the optical 3D scatter plot 74 and the positions of the coils in the FOR of electromagnetic tracking system 24, as found at 301. The mapping provides a registration between the FOR of the electromagnetic tracking system and the FOR of the optical image, and this may be quantified as second transformation matrix M[MAGN-OPT].

At 307, the processor 40 combines the two registrations, produced at 305 and 306, to produce a third registration between the FOR of the electromagnetic tracking system 24 and the FOR of the CT image. The resulting registration may be quantified as a third transformation matrix M[CT-MAGN] and it will be understood that matrix M]CT-MAGN] may be generated from matrices M[MAGN-OPT] and M[CT-OPT].

As noted above, the method of FIG. 3 may be performed on the registration system 20 of FIG. 1 comprising the patient tracker 78. In some embodiments, the registration system 20 does not comprise patient tracker 78. Instead, an additional sensor may be added to the 3D camera 70 to register a 3D image of a head of a patient 22 with magnetic coordinates of the electromagnetic tracking system 24. The additional sensor may determine a location and orientation of the 3D camera 70. The location and orientation of the 3D camera 70 may be used to find registration between the electromagnetic system and CT image, as described in more detail below.

According to the claimed invention, the additional sensor is a tri-axial sensor (TAS). A TAS includes 3 sensors, with 9 transmitters and 2 coils, collects a total of 27 voltage measurements at a single point (3x9=27). The three sensors of the TAS may provide simultaneous measurements in three orthogonal directions. The 3D camera may be tracked and navigated to match the real position (magnetic location and orientation) of the TAS. The location and orientation of the TAS sensor may be determined based on the known magnetic fields that are applied by a magnetic field transmitter. The 3D camera 70 may be tracked and navigated using the TAS sensor, which reads the configuration of the field to search for the location and orientation of the 3D camera 70. The addition of the TAS sensor to the 3D camera 70 provides for the ability to register the 3D camera image with the magnetic coordinates, which enhances the accuracy of the registration of the CT image with the magnetic coordinates.

In other embodiments, the additional sensor is a single-axis sensor (SAS) or dual-axis sensor (DAS). A SAS collects a total of 9 voltage measurements (1x9=9). A DAS collects 18 voltage measurements (2x9=18).

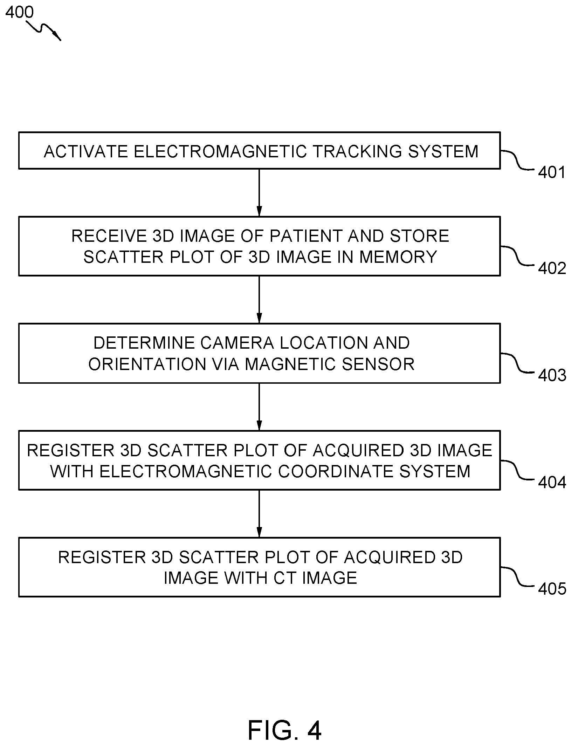

FIG. 4 is a flowchart diagram of a method 400 for executing a registration algorithm according to an embodiment. Method 400 may be performed on the registration system 20 illustrated in FIG. 1, however, method 400 does not require the use of patient tracker 78. Instead of using patient tracker 78, 3D camera 70 further comprises, in addition to the at least one optical sensor, at least one magnetic sensor. This allows for the location and orientation of the 3D camera to be determined and used to register the 3D image with the CT image. According to the claimed invention, the magnetic sensor is a TAS, as discussed above. The TAS may provide simultaneous measurements in three orthogonal directions. In other embodiments, a SAS or DAS may be used instead of a TAS.

As noted above, the 3D camera 70 also comprises at least one optical sensor. In some embodiments, the 3D camera 70 may comprise two, separate optical sensors. The 3D optical image comprises a set of optical voxels, each voxel having three Cartesian coordinates as well as color, typically red, green, blue (RGB) values. The set of optical voxels is herein also termed a 3D scatter plot 74, and the optical voxels of scatter plot 74 are stored in memory 42.

At 401, the electromagnetic tracking system 24 is activated, and the head of patient 22 is placed within region 30 of the system. When the electromagnetic tracking system 24 is activated, magnetic field transmitters 28, which are fixed in position and transmit alternating sinusoidal magnetic fields into a region 30, are placed where the head of the patient 22 is located.

At 402, medical professional 25 activates camera 70 to acquire a 3D optical image of the face of patient 22. The processor 40 stores the acquired 3D image as 3D scatter plot 74 in memory 42.

At 403, the processor 40 may be activated to analyze the voltage measurements of the TAS. The processor 40 is configured to process the signals so as to derive location and orientation values for the sensor. The processor 40 is configured to process the signals so as to derive location and orientation values for the sensor. Other elements of the system 20, including magnetic transmitters 28, are controlled by the system processor 40. Other elements of the system 20, including magnetic transmitters 28, are controlled by the system processor 40. The processor 40 is configured to process the signals so as to derive location and orientation values for the sensor, and thereby the camera. Other elements of the system 20, including magnetic transmitters 28, are controlled by the system processor 40.

In some embodiments, 402 and 403 are performed simultaneously. In further embodiments, time stamps may be taken at both 402 and 403 and cross-referenced to ensure that they match (i.e., to confirm that the time the 3D image of the patient acquired at 402 is the same time at which the camera location and orientation was determined at 403).

At 404, the processor 40 transfers the optical 3D scatter plot 74 to the magnetic coordinate system of the electromagnetic tracking system. The location and orientation of the 3D camera 70 determined at 403 may be used to map the 3D scatter plot 74 to the magnetic coordinates. Any suitable location algorithm may be used to map the 3D scatter plot 74 to the magnetic coordinates. For example, a cloud point matching algorithm, such as ICP or robust point matching, may be used. The mapping may provide a registration between the optical image and the magnetic coordinates.

At 405, the processor may register the 3D image with a CT image of the patient 22. A CT image of the head of patient 22 may be retrieved from memory 42. The processor 40 may analyze the image to identify CT voxels of the stored image corresponding to surface features of the head of the patient 22. The CT voxels may be mapped to the optical voxels of the 3D scatter plot 74. Any suitable algorithm may be used to find the transformation that best maps the optical voxels of the 3D scatter plot 74 to a surface of CT voxels. In some embodiments, an ICP algorithm may be used. This mapping may provide a registration between the optical image and the CT image.

In some embodiments, after completing the registration of the 3D image with the CT image and the 3D image with the magnetic coordinates, the CT image may then be registered with the magnetic coordinates. For example, the processor 40 may combine the registration between the optical image and the magnetic coordinates and the registration between the optical image and the CT image to produce another registration between magnetic coordinates and the CT image. Registering the 3D image with the magnetic coordinates first may enhance the accuracy of the registration of the CT image with the magnetic coordinates.

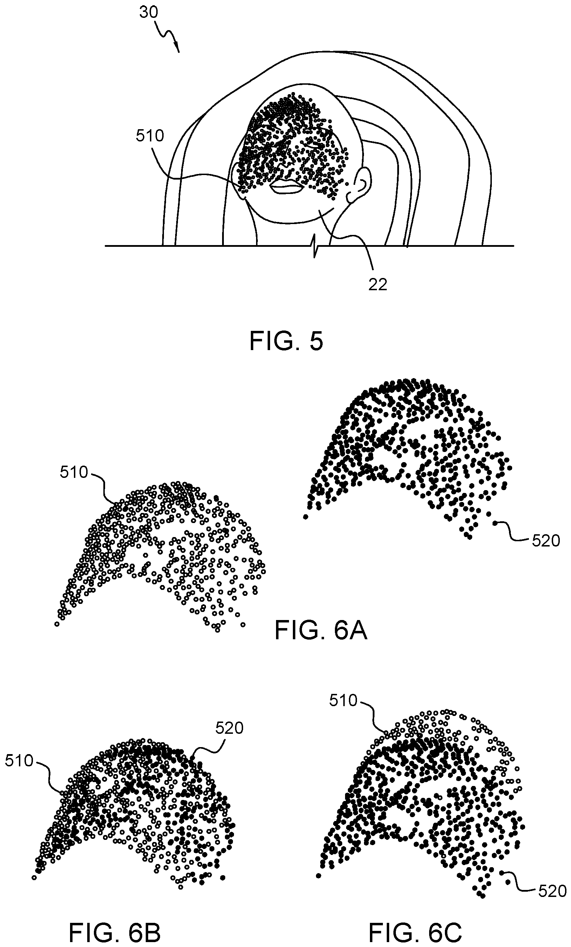

FIG. 5 is a schematic illustration of a 3D scatter plot 510 corresponding to the 3D optical image of a patient 22 positioned with a region 30 of a registration system, according to an embodiment. The registration system may comprise the registration system 20 illustrated in FIG. 1, with the patient tracker 78 being optional. In FIG. 5, the 3D scatter plot 510 of the acquired 3D image is overlaid onto the face of the patient 22 for illustrative purposes.

FIGs. 6A-6C are schematic illustrations of the mapping the 3D scatter plot 510 of the acquired 3D image to the CT coordinate system 520 of the electromagnetic tracking system, according to an embodiment. This mapping may occur in 405 of the method describes with respect to FIG. 4. FIG. 6A illustrates the 3D scatter plot 510 and the CT voxels 520 when they are separate and FIG. FIGs. 6B and 6C are schematic illustrates of the 3D scatter plot 510 being mapping to the CT voxels 520. In some embodiments, the location and orientation of the 3D camera 70 when the 3D image was acquired is used in an algorithm to map the 3D scatter plot to the magnetic coordinates, as discussed above.

It will be appreciated that the embodiments described above are cited by way of example, and that the present disclosure is not limited to what has been particularly shown and described hereinabove. The scope of the present invention is defined by the appended claims.

## Claims

1. A method comprising:
activating an electromagnetic tracking system (24) positioned around a head of a patient;
receiving a three-dimensional, 3D, image of a surface of the head of the patient and storing the 3D image as a scatter plot (74) in a memory (42), wherein the 3D image is acquired using a 3D camera comprising at least one optical sensor, **characterised by** the 3D camera further comprising at least one magnetic sensor, wherein the at least one magnetic sensor is a tri-axial sensor, TAS, and by the method further comprising:
determining a location and an orientation of the 3D camera when the 3D image was acquired via the at least one magnetic sensor based on known magnetic fields applied by one or more transmitters of the electromagnetic tracking system;
registering the scatter plot to magnetic coordinates of the electromagnetic tracking system using the determined location and orientation of the 3D camera, wherein the registered scatter plot is stored in the memory.

2. The method of claim 1, further comprising registering the received 3D image of the patient with a computerized tomography, CT, image of the patient.

3. The method of claim 2, further comprising registering the CT image with the magnetic coordinates.

4. A system (20) comprising
an electromagnetic tracking system (24) comprising one or more transmitters (28) configured to apply magnetic fields;
a 3D camera (70) comprising at least one optical sensor;
a memory (42) configured to store a 3D image of a surface of a head of a patient acquired by the 3D camera as a scatter plot (74); and
a processor (40),
**characterized by** the 3D camera further comprising at least one magnetic sensor, wherein the at least one magnetic sensor is a tri-axial sensor, TAS, and the processor being configured to:
activate the electromagnetic tracking system;
retrieve the scatter plot from the memory;
determine a location and an orientation of the 3D camera when the 3D camera acquired the 3D image via the at least one magnetic sensor based on known magnetic fields applied by the one or more transmitters of the electromagnetic tracking system; and
register the scatter plot to magnetic coordinates of the electromagnetic tracking system;
the memory further configured to store the registered scatter plot.

5. The system of claim 4, wherein the processor is further configured to register a computerized tomography, CT, image of the patient with the 3D image.

6. The system of claim 5, wherein the CT image is registered with the magnetic coordinates using an Iterative Closest Point, ICP, algorithm.

7. The system of claim 6, wherein the processor is further configured to register the CT image with the magnetic coordinates.

8. A non-transitory computer readable storage medium in which program instructions are stored, which, when read by a processor, cause the processor to:
activate an electromagnetic tracking system comprising one or more transmitters (28) configured to apply magnetic fields;
retrieve a scatter plot of a 3D image of a surface of a head of a patient, wherein the 3D image is acquired by a 3D camera comprising at least one optical sensor and at least one magnetic sensor, wherein the at least one magnetic sensor is a tri-axial sensor, TAS;
determine a location and an orientation of the 3D camera when the 3D image was acquired via the at least one magnetic sensor based on known magnetic fields applied by the one or more transmitters of the electromagnetic tracking system; and
register the scatter plot to magnetic coordinates of the electromagnetic tracking system.

9. The non-transitory computer readable storage medium of claim 8, wherein the stored program instructions, when read by a processor, further cause the processor to register a computerized tomography, CT, image with the 3D image.

10. The non-transitory computer readable storage medium of claim 9, wherein the stored program instructions, when read by a processor, further cause the processor to register the CT image with the magnetic coordinates.

## Patentansprüche

1. Verfahren, umfassend:
Aktivieren eines elektromagnetischen Verfolgungssystems (24), das um einen Kopf eines Patienten herum positioniert ist;
Empfangen eines dreidimensionalen Bildes, 3D-Bildes, einer Oberfläche des Kopfes des Patienten und Speichern des 3D-Bildes als ein Streuungsdiagramm (74) in einem Speicher (42), wobei das 3D-Bild unter Verwendung einer 3D-Kamera aufgenommen wird, umfassend mindestens einen optischen Sensor, **gekennzeichnet dadurch, dass** die 3D-Kamera ferner mindestens einen magnetischen Sensor umfasst, wobei der mindestens eine magnetische Sensor ein dreiachsiger Sensor, TAS, ist, und dass das Verfahren ferner umfasst:
Bestimmen eines Standorts und einer Ausrichtung der 3D-Kamera, wenn das 3D-Bild über den mindestens einen magnetischen Sensor aufgenommen wurde, basierend auf bekannten magnetischen Feldern, die durch einen oder mehrere Sender des elektromagnetischen Verfolgungssystems angelegt werden;
Registrieren des Streuungsdiagramms in magnetischen Koordinaten des elektromagnetischen Verfolgungssystems unter Verwendung der bestimmten Position und der bestimmten Ausrichtung der 3D-Kamera, wobei das registrierte Streuungsdiagramm in dem Speicher gespeichert wird.

2. Verfahren nach Anspruch 1, ferner umfassend das Registrieren des empfangenen 3D-Bildes des Patienten mit einem Computertomographie-Bild, CT-Bild, des Patienten.

3. Verfahren nach Anspruch 2, ferner umfassend das Registrieren des CT-Bildes mit den magnetischen Koordinaten.

4. System (20), umfassend
ein elektromagnetisches Verfolgungssystem (24), umfassend einen oder mehrere Sender (28), die konfiguriert sind, um magnetische Felder anzulegen;
eine 3D-Kamera (70), umfassend mindestens einen optischen Sensor;
einen Speicher (42), der konfiguriert ist, um ein 3D-Bild einer Oberfläche eines Kopfes eines Patienten, das durch die 3D-Kamera aufgenommen wird, als ein Streuungsdiagramm (74) zu speichern; und
einen Prozessor (40),
**dadurch gekennzeichnet, dass** die 3D-Kamera ferner mindestens einen magnetischen Sensor umfasst, wobei der mindestens eine magnetische Sensor ein dreiachsiger Sensor, TAS, ist, und der Prozessor konfiguriert ist zum:
Aktivieren des elektromagnetischen Verfolgungssystems;
Abrufen des Streuungsdiagramms von dem Speicher;
Bestimmen eines Standorts und einer Ausrichtung der 3D-Kamera, wenn die 3D-Kamera das 3D-Bild über den mindestens einen magnetischen Sensor aufnimmt, basierend auf bekannten magnetischen Feldern, die durch den einen oder die mehreren Sender des elektromagnetischen Verfolgungssystems angelegt werden; und
Registrieren des Streuungsdiagramms in den magnetischen Koordinaten des elektromagnetischen Verfolgungssystems;
wobei der Speicher ferner konfiguriert ist, um das registrierte Streuungsdiagramm zu speichern.

5. System nach Anspruch 4, wobei der Prozessor ferner konfiguriert ist, um ein Computertomographie-Bild, CT-Bild, des Patienten mit dem 3D-Bild zu registrieren.

6. System nach Anspruch 5, wobei das CT-Bild unter Verwendung eines Iterative-Closest-Point-Algorithmus, ICP-Algorithmus, mit den magnetischen Koordinaten registriert wird.

7. System nach Anspruch 6, wobei der Prozessor ferner konfiguriert ist, um das CT-Bild mit den magnetischen Koordinaten zu registrieren.

8. Nicht flüchtiges, computerlesbares Speichermedium, in dem Programmanweisungen gespeichert sind, die, wenn sie durch einen Prozessor gelesen werden, den Prozessor veranlassen zum:
Aktivieren eines elektromagnetischen Verfolgungssystems, umfassend einen oder mehrere Sender (28), die konfiguriert sind, um magnetische Felder anzulegen;
Abrufen eines Streuungsdiagramms eines 3D-Bildes einer Oberfläche eines Kopfes eines Patienten, wobei das 3D-Bild durch eine 3D-Kamera aufgenommen wird, umfassend mindestens einen optischen Sensor und mindestens einen magnetischen Sensor, wobei der mindestens eine magnetische Sensor ein dreiachsiger Sensor, TAS, ist;
Bestimmen eines Standorts und einer Ausrichtung der 3D-Kamera, wenn das 3D-Bild über den mindestens einen magnetischen Sensor aufgenommen wurde, basierend auf bekannten magnetischen Feldern, die durch den einen oder die mehreren Sender des elektromagnetischen Verfolgungssystems angelegt werden; und
Registrieren des Streuungsdiagramms in den magnetischen Koordinaten des elektromagnetischen Verfolgungssystems.

9. Nicht flüchtiges, computerlesbares Speichermedium nach Anspruch 8, wobei die gespeicherten Programmanweisungen, wenn sie durch einen Prozessor gelesen werden, den Prozessor ferner veranlassen, ein Computertomographie-Bild, CT-Bild, mit dem 3D-Bild zu registrieren.

10. Nicht flüchtiges, computerlesbares Speichermedium nach Anspruch 9, wobei die gespeicherten Programmanweisungen, wenn sie durch einen Prozessor gelesen werden, den Prozessor ferner veranlassen, das CT-Bild mit den magnetischen Koordinaten zu registrieren.

## Revendications

1. Procédé comprenant :
l'activation d'un système de suivi électromagnétique (24) positionné autour d'une tête d'un patient ;
la réception d'une image tridimensionnelle, 3D, d'une surface de la tête du patient et le stockage de l'image 3D sous forme de diagramme de dispersion (74) dans une mémoire (42), dans lequel l'image 3D est acquise à l'aide d'une caméra 3D comprenant au moins un capteur optique, **caractérisé par** la caméra 3D comprenant en outre au moins un capteur magnétique, dans lequel l'au moins un capteur magnétique est un capteur tri-axial, TAS, et **par** le procédé comprenant en outre :
la détermination d'un emplacement et d'une orientation de la caméra 3D lorsque l'image 3D a été acquise par l'intermédiaire de l'au moins un capteur magnétique, sur la base de champs magnétiques connus appliqués par un ou plusieurs émetteurs du système de suivi électromagnétique ;
l'enregistrement du diagramme de dispersion en coordonnées magnétiques du système de suivi électromagnétique à l'aide de l'emplacement et de l'orientation déterminés de la caméra 3D, dans lequel le diagramme de dispersion enregistré est stocké dans la mémoire.

2. Procédé selon la revendication 1, comprenant en outre l'enregistrement de l'image 3D reçue du patient avec une image de tomodensitométrie, TDM, du patient.

3. Procédé selon la revendication 2, comprenant en outre l'enregistrement de l'image TDM avec les coordonnées magnétiques.

4. Système (20) comprenant
un système de suivi électromagnétique (24) comprenant un ou plusieurs émetteurs (28) configurés pour appliquer des champs magnétiques ;
une caméra 3D (70) comprenant au moins un capteur optique ;
une mémoire (42) configurée pour stocker une image 3D d'une surface d'une tête d'un patient acquise par la caméra 3D sous forme de diagramme de dispersion (74) ; et
un processeur (40),
**caractérisé par** la caméra 3D comprenant en outre au moins un capteur magnétique, dans lequel l'au moins un capteur magnétique est un capteur tri-axial, TAS, et le processeur étant configuré pour :
activer le système de suivi électromagnétique ;
récupérer le diagramme de dispersion de la mémoire ;
déterminer un emplacement et une orientation de la caméra 3D lorsque la caméra 3D a acquis l'image 3D par l'intermédiaire de l'au moins un capteur magnétique, sur la base de champs magnétiques connus appliqués par le ou les émetteurs du système de suivi électromagnétique ; et
enregistrer le diagramme de dispersion en coordonnées magnétiques du système de suivi électromagnétique ;
la mémoire est en outre configurée pour stocker le diagramme de dispersion enregistré.

5. Système selon la revendication 4, dans lequel le processeur est en outre configuré pour enregistrer une image de tomodensitométrie, TDM, du patient avec l'image 3D.

6. Système selon la revendication 5, dans lequel l'image TDM est enregistrée avec les coordonnées magnétiques à l'aide d'un algorithme de point itératif le plus proche, ICP.

7. Système selon la revendication 6, dans lequel le processeur est en outre configuré pour enregistrer l'image TDM avec les coordonnées magnétiques.

8. Support de stockage non transitoire lisible par ordinateur dans lequel sont stockées des instructions de programme, qui, lorsqu'elles sont lues par un processeur, amènent le processeur à :
activer un système de suivi électromagnétique comprenant un ou plusieurs émetteurs (28) configurés pour appliquer des champs magnétiques ;
récupérer un diagramme de diffusion d'une image 3D d'une surface d'une tête d'un patient, dans lequel l'image 3D est acquise par une caméra 3D comprenant au moins un capteur optique et au moins un capteur magnétique, dans lequel l'au moins capteur magnétique est un capteur tri-axial, TAS ;
déterminer un emplacement et une orientation de la caméra 3D lorsque l'image 3D a été acquise par l'intermédiaire de l'au moins un capteur magnétique, sur la base de champs magnétiques connus appliqués par le ou les émetteurs du système de suivi électromagnétique ; et
enregistrer le diagramme de dispersion en coordonnées magnétiques du système de suivi électromagnétique.

9. Support de stockage non transitoire lisible par ordinateur selon la revendication 8, dans lequel les instructions de programme stockées, lorsqu'elles sont lues par un processeur, amènent en outre le processeur à enregistrer une image de tomodensitométrie, TDM avec l'image 3D.

10. Support de stockage non transitoire lisible par ordinateur selon la revendication 9, dans lequel les instructions de programme stockées, lorsqu'elles sont lues par un processeur, amènent en outre le processeur à enregistrer l'image TDM avec les coordonnées magnétiques.
